(19) Europäisches Patentamt
European Patent Office
Office européen des brevets

(11) **EP 1 456 412 B1**

(12) **EUROPEAN PATENT SPECIFICATION**

(45) Date of publication and mention
of the grant of the patent:
**02.11.2011 Bulletin 2011/44**

(51) Int Cl.:
**C12Q 1/68** (2006.01)

(21) Application number: **02788208.3**

(22) Date of filing: **20.12.2002**

(86) International application number:
**PCT/GB2002/005837**

(87) International publication number:
**WO 2003/056035 (10.07.2003 Gazette 2003/28)**

(54) **IMPROVEMENTS IN AND RELATING TO INTERPRETING DNA**

VERBESSERUNGEN BEI UND IN BEZUG AUF DIE INTERPRETATION VON DNA

AMELIORATIONS APPORTEES A L'INTERPRETATION DE L'ADN

(84) Designated Contracting States:
**AT BE BG CH CY CZ DE DK EE ES FI FR GB GR IE IT LI LU MC NL PT SE SI SK TR**

(30) Priority: **21.12.2001 GB 0130674**

(43) Date of publication of application:
**15.09.2004 Bulletin 2004/38**

(73) Proprietor: **Forensic Science Service Ltd**
**London EC3N 2AA (GB)**

(72) Inventors:
  • **FOREMAN, Lindsey,**
    **The Forensic Science Service**
    **London SE1 7LP (GB)**
  • **EVETT, Ian,**
    **The Forensic Science Service**
    **London SE1 7LP (GB)**
  • **POPE, Susan,**
    **The Forensic Science Service**
    **London SE1 7LP (GB)**
  • **BUCKLETON, John,**
    **ESR,**
    **Mt. Albert Science Centre**
    **Mt. Albert,**
    **Auckland (NZ)**
  • **CURRAN, James,**
    **University of Waikato**
    **Hamilton (NZ)**
  • **TRIGGS, Christopher,**
    **University of Auckland**
    **Auckland (NZ)**

(74) Representative: **Pawlyn, Anthony Neil**
**Urquhart-Dykes & Lord LLP**
**Tower North Central**
**Merrion Way**
**Leeds LS2 8PA (GB)**

(56) References cited:
**WO-A-01/46466     WO-A-01/79541**

• **CLAYTON T M ET AL: "ANALYSIS AND INTERPRETATION OF MIXED FORENSIC STAINS USING DNA STR PROFILING" FORENSIC SCIENCE INTERNATIONAL, ELSEVIER SCIENTIFIC PUBLISHERS IRELAND LTD, IE, vol. 91, no. 1, 9 January 1998 (1998-01-09), pages 55-70, XP001013202 ISSN: 0379-0738**
• **GILL P ET AL: "INTERPRETING SIMPLE STR MIXTURES USING ALLELE PEAK AREAS" FORENSIC SCIENCE INTERNATIONAL, ELSEVIER SCIENTIFIC PUBLISHERS IRELAND LTD, IE, vol. 91, no. 1, 1998, pages 41-53, XP001012655 ISSN: 0379-0738**
• **GILL P ET AL: "AUTOMATED SHORT TANDEM REPEAT (STR) ANALYSIS IN FORENSIC CASEWORK - A STRATEGY FOR THE FUTURE" ELECTROPHORESIS, WEINHEIM, DE, vol. 16, no. 9, 1995, pages 1543-1552, XP001027034 ISSN: 0173-0835**
• **GILL P ET AL: "DEVELOPMENT OF GUIDELINES TO DESIGNATE ALLELES USING AN STR MULTIPLEX SYSTEM" FORENSIC SCIENCE INTERNATIONAL, ELSEVIER SCIENTIFIC PUBLISHERS IRELAND LTD, IE, vol. 89, no. 3, 1997, pages 185-197, XP001027298 ISSN: 0379-0738**

**(Cont. next page)**

- **WEIR B S ET AL: "INTERPRETING DNA MIXTURES" JOURNAL OF FORENSIC SCIENCES, CALLAGHAN AND CO, CHICAGO, IL, US, vol. 42, no. 2, March 1997 (1997-03), pages 213-222, XP001012657 ISSN: 0022-1198**
- **GILL P ET AL: "INTERPRETATION OF SIMPLE MIXTURES OF WHEN ARTEFACTS SUCH AS STUTTERS ARE PRESENT - WITH SPECIAL REFERENCE TO MULTIPLEX STRS USED BY THE FORSENIC SCIENCE SERVICE" FORENSIC SCIENCE INTERNATIONAL, ELSEVIER SCIENTIFIC PUBLISHERS IRELAND LTD, IE, vol. 95, no. 3, 12 August 1998 (1998-08-12), pages 213-224, XP001027029 ISSN: 0379-0738**

**Description**

This invention concerns improvements in and relating to interpreting DNA, particularly, but not exclusively, in relation to interpreting DNA mixtures.

In an increasing number of cases, DNA profiles of samples containing DNA reveal more than two alleles at one or more loci under consideration. As a consequence these results are deemed to arise from mixtures, and they must be considered accordingly in any subsequent evaluation.

It is desirable to be able to establish whether or not, and more preferably establish a likelihood ratio, that the mixture arose from a particular scenario. The particular scenario may vary from case to case, but a not infrequent situation is comparing the scenario that the mixture arose from the victim and a suspect with a scenario in which the mixture arose from the victim and another unknown individual.

Presently mixtures are analysed by extremely experienced senior forensic scientists to assess whether the mixture result measured is consistent with the suggested scenario or scenarios. Whilst carried out as effectively as possible, the consideration is inevitably subjective and is unsuited to use by less experienced forensic scientists. The process of interpretation is based on personal knowledge and renders such mixtures analysis unsuited for automation through the use of expert systems and the like.

When a sample of DNA believed to be from a single source is profiled, for instance for entry onto a database, the experimentally derived profile obtained is considered and a determination is made as to the genotype behind it. That determination of the genotype is usually based on the application of one or more rules. It would be useful to be able to rigorously confirm that the determined genotype was the appropriate one.

Examples of techniques where results are obtained from a sample and are then interpreted to reveal the allele identities associated with the individual(s) behind the sample, hence giving their genotypes and with their genotypes then be compared with other genotypes for a match are to be found in the prior art, including Gill et al., "Interpretation of simple mixtures of when artefacts such as stutters are present - with special reference to multiplex STRs used by the Forensic Science Service ", Forensic Science International 95 (1998) 213-224.

Clayton et al "Analysis And Interpretation Of Mixed Forensic Stains Using DNA STR Profiling", Forensic Science International, Elsiever Scientific Publishers Ireland Ltd, vol 91, no 1, pages 55-70 provides a method for establishing the genotype of a DNA sample, the method including: a) analysing the sample to produce a data profile for the sample for a locus. That is then analysed and interpreted to give a profile or genotype which is than comparing that with a reference genotype obtained by analysing a sample taken in controlled circumstances from an individual.

WO 01/007954 provides a similar approach and works from samples through analysis and interpretation to genotypes which are then compared.

The problem with such approaches is that potentially useful information is excluded from the record in moving from an experimental result to a genotype through interpretation and the range of results which give a genotype fit for recordal on a database are limited.

The present invention has amongst its aims to provide an improved technique for considering mixtures of DNA. The present invention has amongst its aims to provide a method of interpretation of mixtures which is suitable for use in an expert system. The present invention has amongst its aims to provide a method which is suitable for use in an automated process. The present invention has amongst its aims to validate the genotype determined for an experimentally derived profile.

According to a first aspect of the invention we provide a method for providing an indication of the likelihood of a data profile being given by a suggested genotype for a DNA sample, the method including:

a) analysing the sample to produce a data profile for the sample for a locus, the data profile including a peak height and/or peak area at one or more allele identities;
b) proposing a suggested genotype;
the method being **characterised in that** the method also includes:
c) generating a first stage profile for the locus for the suggested genotype, the first stage profile including a peak height and/or peak area at each allele identity in the suggested genotype;
d) adjusting the first stage profile to account for one or more factors to give a simulation profile, the one or more factors including at least one of preferential amplification and/or stutter and/or allele dropout and/or allele drop in and/or stochastic components and/or noise, the adjustment altering the peak height and/or peak areas and/or peak position and/or peak distribution for one or more of the allele identities;
e) comparing the data profile and the simulation profile to provide an indication of the likelihood of the data profile being given by the suggested genotype, the comparing of the data profile and the simulation profile being a statistically based measure of the level of match or correspondence between the data profile and the simulation profile;
and wherein, the generation of the first stage profile and the adjustments to the first stage profile and the comparison of the data profile and the simulation method are performed by an expert system or by an automated process.

Preferably the method is repeated for a plurality of loci and/or for a plurality of proposed genotypes.

The method may provide a method for providing information on the likelihood of the results of the analysis of a DNA sample given one or more particular genotypes, the DNA sample arising from a plurality of sources, the method including:- analysing the sample to produce a data profile for the sample for a plurality of loci;

proposing a plurality of suggested genotypes for the plurality of sources;

generating a first stage profile for the locus for one of the suggested genotypes;

adjusting the first stage profile to account for one or more factors to give a simulation profile for the one of the suggested genotypes;

comparing the data profile and the simulation profile for the one of the suggested genotypes to provide an indication of the likelihood of the data profile given the one suggested genotype;

repeating these stages for each of the other suggested genotypes.

The first and/or second aspects of the invention may include any of the following features, options or possibilities.

The method may give an indication as to the likelihood of the data profile given one or more suggested genotypes as an indication as the the likelihood of the data profile for a suggested genotype for each of one or more suggested genotypes. The indication may be a possible or not possible indication for that data profile given a particular suggested genotype. The indication may be a likelihood ratio relating the likelihood of the data profile given one or more first suggested genotypes to the likelihood of the data profile given one or more second suggested genotypes. The first genotype or genotypes may be suggested by the prosecution in a case. The second genotype or genotypes may be suggested by the defence in a case. The first genotype or genotypes may be the victim of a crime and a suspect. The second genotype or genotypes may be all other genotypes than those included in the first genotype or genotypes. The second genotype or genotypes may be the victim of the crime and all other genotypes other than that of the suspect.

The indication of the likelihood of the data profile given the suggested genotype may be used to propose one or more search genotypes from amongst the suggested genotypes. The one or more search genotypes may be those suggested genotypes for which the indication of the likelihood of the data profile given that suggested genotype is the highest or amongst the highest. The search genotypes may be the X most likely, where X is an integer and is 100 or less, preferably 50 or less, more preferably 20 or less, ideally 10 or less. The search genotype may be the most likely suggested genotype. The one or more search genotypes may be those suggested genotypes for which the indication of likelihood is above a predetermined level. The search genotypes may be rated according to their indication of likelihood.

The one or more search genotypes may be recorded or otherwise entered into a database, particularly a database of genotypes. The record for each of the suggested genotypes may detail the origins of that genotype. The database may contain records as genotypes and/or as profiles. The search genotypes may be recorded or entered as genotypes or as profiles. The database may be a database of records of previously analysed DNA samples. The database may be a database of genotypes of known people. The database may be a database of genotypes from crime scenes and/or from unsolved crimes. The database may be an intelligence database. The previous samples may be from known and/or unknown sources. The previous samples may be mixtures. The previous samples may be mixtures from one or more known and one or more unknown sources. may be mixtures from one or more known and one or more unknown sources. The previous samples may have been taken from an individual and/or a location and/or an item. Preferably, once recorded or otherwise entered into the database, the one or more search genotypes can themselves be searched against as database genotypes in future searches.

The one or more search genotypes may be searched against the contents of a database, particularly a database of genotypes. The one or more search genotypes may be searched against the database separately from one another. The search may be for one or more database genotypes that match the one or more search genotypes. The presence of a match may be considered in profile and/or genotype form. The presence of a match may be considered using a statistical tool. A match may be indicated in terms of a yes/no type indication. Preferably only those database genotypes for which a match with the search genotype is determined are indicated to the user. The match may be indicated by presenting the indication of the likelihood of the match, and particularly the value therefore. The search process may produce one or more indications of a match. The one or more indications of a match may be listed and are preferably ranked within the list. Matches with respect to different search genotypes are preferably indicated separately from one another.

The presence of a match between a search genotype, and a database genotype could be used as an indication of a link between the search genotype and the database genotype. The link may be that the source of the search genotype and the database genotype were the same person. The link may be that the known source of the database genotype is a suspect and/or that the known source was the source of the analysed sample which gave rise to the suggested genotype. The presence of a match may form part of the evidence against a suspect. The presence of a match may assist the direction and/or conduct of further enquiries, for instance by law enforcement authorities.

The one or more search genotypes may be recorded or otherwise entered into the database as part of the searching process. Preferably in such a case, the one or more search genotypes can themselves be searched against as database genotypes in future searches.

Alternatively, the one or more search genotypes may be subjected to the searching process, without being recorded or otherwise entered into the database, particularly to form database genotypes for later searches.

One or more search genotypes from a group of search genotypes may be recorded or otherwise entered into a database, whilst one or more other search genotypes from the group of search genotypes may not be recorded or otherwise entered on a database. All the search genotypes in the group may be searched against the database for matches with database genotypes. Preferably the search genotypes included in the group are those having an indication of likelihood above a first level. Preferably those search genotypes which are recorded or otherwise entered in the database are those having an indication of likelihood above a second level. Preferably the second level is higher than the first level. Preferably those search genotypes which are not recorded or otherwise entered in the database, but are searched against the database are those having a likelihood indication above the first level, but below the second level.

The potential sources may include one or more known individuals, for instance a victim of a crime. The potential sources may include one or more suspects for a crime. The potential sources may be from one or more unknown individuals. DNA profiles for one or more of the potential sources may exist, particularly profiles generated from single source DNA samples.

The sample may be taken from an individual and/or a location and/or an item.

The plurality of sources may be greater than two. The number of sources may be unknown. The relative contribution of each source to the sample may be unknown. The relative contributions may be equal to one another or may be different to one another. The likelihood of the data profile given a particular number of sources contributing may be established as part of the method.

The data profile may be produced by profiling after amplification, particularly using PCR based amplification. The sample may be profiled using slab gel electrophoresis and/or capillary gel electrophoresis. The data profile may be generated as part of an automated process. The data profile may be determined as part of an automated process.

The data profile may be represented graphically. The data profile may be represented numerically. Distinct peak or peak area contributions for allele identities at each locus may be considered.

The data profile may include a profile from more than one locus. At least 6 and more preferably at least 8 and ideally at least 10 loci may be considered in the acquisition of the data profile.

The suggested genotype may be proposed manually, for instance by an operator. The suggested genotype may be suggested automatically. The suggested genotype may be suggested by an expert system. The suggested genotype may be suggested by an automated system.

The suggested genotype may be suggested for one locus. Preferably the suggested genotype includes a suggestion for each locus under consideration, ideally the loci considered in the data profile. The first stage profile and/or the one or more factors may be applied separately to each locus.

A separate method of investigation or method of providing information may be applied to each locus. The results from the different loci may be combined to give am overall indication. The method may suggest a genotype for each of at least 6 loci, more preferably at least 8 loci and ideally at least 10 loci.

The suggested genotype may be the only genotype considered by the method. A plurality of genotypes may be considered by the method. All possible genotypes may be considered by the method.

The suggested genotype may include specification of the number of contributing sources. The suggested genotype may include specification of two of the allele identities at a locus to a source and ideally two of the allele identities to each of the sources.

Preferably the same number of contributing sources is specified for all loci under consideration.

In one embodiment of the invention a majority proportion of all the possible genotypes for the number of sources may be considered. The proportion may be at least 70%, possible at least 80% and potentially all of the possible genotypes.

In another embodiment of the invention a selection of genotypes from amongst all the possible genotypes is made and these are compared with the data profile. The selection may be less than 25%, more preferably less than 10%, of the total number of possible genotypes.

The generation of the first stage profile is preferably performed in the same format as used by the data profile. The format may be graphical. The format may be numerical. The peak heights and/or peak areas may be the same for each allele identity in the base profile. A first stage profile is preferably generated for each locus.

The adjustment may take the first stage profile, potentially through one or more intervening profiles, to the simulation profile. Preferably the simulation profile is an anticipation of the data profile which would be expected to occur for that genotype in practice. Preferably the adjustment simulates the experimentally determined profile expected for a suggested genotype. Preferably the adjustment accounts for a plurality of factors. The adjustment may be made in a single step or the adjustment for separate factors may be made in a series of separate steps.

The adjustment may be based on a model of the factor's impact, particularly its impact on the peak height and/or peak area and/or peak position and/or peak distribution. One or more of the models may be theoretically determined. Preferably one or more of the models are derived from experimental evidence, such as prior investigations of the factor's impact. The model may be different for each factor. The model may be a distribution from which an adjustment term is taken at random. The distribution may be a normal distribution. The model may be a shaped distribution, particularly defined be a beta parameter.

The adjustment for one or more of the separate factors preferably includes within the adjustment an account of the variable effect of the factor on different occasions. The adjustment for one or more of the separate factors preferably includes within the adjustment an account of the random nature of the extent of the effect of the factor on different occasions. Preferably the adjustment for the one or more separate factors does not apply an adjustment which has a fixed value for each occasion.

Particularly for preferential amplification a normal distribution is preferred as the model. In the case of preferential amplification preferably the normal distribution is used to generate number z, where z is the random number generated for the peak area of the lighter peak in a given contributor's genotype. Preferably the mean, $\mu$ and standard deviation for the normal distribution at one or more and preferably each loci is estimated by computing a value $z_j$ for the $j^{th}$ heterozygote in the sample, the value $z_j$ representing the proportion of the total normalised peak area corresponding to the lighter of the heterozygote peaks.

Particularly for allele drop out, a zero peak height is applied to one or more of the peaks. Allele drop out can be modelled using the cumulative distribution function of a Normal distribution.

Particularly for stutter, a shaped distribution, particularly defined by a Beta parameter, is preferred as the model. In the case of stutter preferably the shaped distribution is defined by the Beta parameter, where a and b are inferred from prior experimentally determined information. Preferably the mean and standard deviation for the prior information are deemed to give the shape parameters, a and b, by the equations :-Mean = a / (a+b) and Variance = (standard deviation)$^2$ = ab / [(a+b+1)(a+b)2].

Particularly for noise, a shaped distribution, particularly defined by a Gamma distribution, is preferred as the model. The Gamma distribution may be defined, ideally at each allele position, by Ga($\gamma,\delta$), where $\gamma$ is the shape parameter and $\delta$ is the scale parameter. Preferably $\gamma$ and $\delta$ are determined from prior experimentally determined information. The model may assume noise acts independently at each of the peak positions. The model may assume that noise acts in an additive manner to the peak areas. The model may assume that noise depends on the total peak area observed in the data profile.

Preferably the accounting for one or more of the above factors adjusts the first stage profile to a modelled profile.

The modelled profile may be adjusted to give the simulation profile by making an adjustment to account for the relative contributions of the two or more sources to the sample. The adjustment for the relative contributions of the sources may apply a weighting to the peak heights and/or peak areas. The weighting may be expressed as a decimal fraction of the contribution from a source and preferably a decimal fraction is allocated to each source. The decimal fractions preferably total to 1. The adjustment may be made by multiplying the peak height and/or peak area by the weighting, preferably a decimal fraction. Preferably the weighting for a given source's contribution is applied to that sources allele identities.

The relative contributions of the contributing sources may be accounted for in the modelled profile to simulation profile adjustment stage. Alternatively the suggested genotype could include specification of the relative contributions of the contributing sources or the relative, particularly when the first stage profile is generated. The specification may provide relative contributions at one of a discrete number of possibilities. The number of possibilities may be 10 or less for each source. The same relative contributions from the sources may be specified for each locus.

The comparing of the data profile and the simulation profile may be a statistically based measure of the level of match or correspondence between the data profile and the simulation profile.

In one embodiment the comparing may use a Monte Carlo based simulations. Preferably the data profile and simulation profile are each deemed to define a data vector and the relative separation of the two data vectors can be quantified. The comparison of the data profile data vector and the simulation profile data vector may give rise to an expression of the comparison in terms of a distance separation, preferably expressed in Euclidean distance. More likely matches may be deemed to be those within a distance Q of the measured data vector. Q may be arbitrarily set. Preferably a number of matches x within distance Q arises as a result out of a total set of N attempts. Preferably a proxy for the probability density function is then used. The proxy used may be Proxy = x/N. A proxy may be calculated across all the loci.

In one embodiment the comparison may be made using Markov Chain Monte Carlo based simulations. The Markov Chain Monte Carlo simulation may select those suggested genotypes to be considered out of all the possible genotypes. The selections may include a selection of the relative contributions of the two or more sources to the sample which are used in the generation of the simulation profile. The Markov Chain Monte Carlo simulation may commence with a first genotype which is a strong candidate The Markov Chain Monte Carlo simulation may miss out a number of genotypes and/or relative contribution forms between a simulation profile and the next simulation profile. Preferably the steps between simulation profiles are smaller where the simulation profile and data profile are highly matched than where they are poorly matched. Highly and/or poorly matched occasions may be those above a threshold and those below it respectively.

The indication may be a likelihood ratio and/or a probability. The indication may include an assessment of the

function $\int p(d \mid A_i, w, H_p) p(w)dw$ and/or $\int p(d \mid A_i, w, H) p(w)dw$ and/or $\int LR(w) p(w)dw$.

Preferably the determination of the data profile and/or the suggestion of the suggested genotype and/or the provision of the first stage profile and/or the adjustment to the modelled profile and/or the adjustment to the simulation profile and/or the comparison and/or the indication are performed by an expert system and ideally by an automated process. Preferably all of the steps are provided by an expert system and ideally by an automated process. Preferably no expert input is required during application of the method to a sample.

The method may further provide steps establishing the genotype of a DNA sample, the method including analysing the sample to produce a data profile for the sample for a locus;
proposing a suggested genotype;
generating a first stage profile for the locus for the suggested genotype;
adjusting the first stage profile to account for one or more factors to give a simulation profile;
comparing the data profile and the simulation profile to provide an indication of the likelihood of the data profile given the suggested genotype.

Preferably the method is repeated for a plurality of loci and/or for a plurality of proposed genotypes.

The method may further provide steps for establishing the genotype of a DNA sample, the method including analysing the sample to produce a data profile for the sample for a plurality of loci;
proposing a plurality of suggested genotypes for the plurality of sources;
generating a first stage profile for the locus for one of the suggested genotypes;
adjusting the first stage profile to account for one or more factors to give a simulation profile for the one of the suggested genotypes;
comparing the data profile and the simulation profile for the one of the suggested genotypes to provide an indication of the likelihood of the data profile given the one suggested genotype;
repeating these stages for each of the other suggested genotypes.

The indication of the likelihood of the data profile given the suggested genotype is preferably used to determine the genotype assigned to that sample, for instance for future search and other consideration purposes. Preferably where the indication of likelihood meets predetermined criteria that suggested genotype is accepted as representing that sample. The predetermined criteria may be an indication above a certain level. The predetermined criteria may be that the suggested genotype is the only likely genotype, for instance by virtue of the level of the indication of likelihood and/or the separate in that indication compared with the indications for the other suggested genotypes.

The indication of the likelihood may indicate that the data profile is more likely given a suggested genotype based on the sample being a mixture than a suggested genotype based on a single source sample. The indication of likelihood may provide an indicate that the data profile given a suggested genotype based on the sample being a mixture is a possibility. The method may provide an indication that the sample could be a mixture, instead of a single source sample.

The indication of the likelihood of the data profile given the suggested genotype may be used to propose one or more search genotypes from amongst the suggested genotypes. The search genotype may be the most likely suggested genotype. The one or more search genotypes may be those suggested genotypes for which the indication of likelihood is above a predetermined level. The search genotypes may be rated according to their indication of likelihood.

The one or more search genotypes may be recorded or otherwise entered into a database, particularly a database of genotypes. The record for each of the suggested genotypes may detail the origins of that genotype. Preferably, once recorded or otherwise entered into the database, the one or more search genotypes can themselves be searched against as database genotypes in future searches.

The one or more search genotypes may be searched against the contents of a database, particularly a database of genotypes. The search may be for one or more database genotypes that match the one or more search genotypes. The one or more indications of a match may be listed and are preferably ranked within the list.

The presence of a match between a search genotype, and a database genotype could be used as an indication of a link between the search genotype and the database genotype. The link may be that source of the search genotype and the database genotype were the same person. The link may be that the known source of the database genotype is a suspect and/or that the known source was the source of the analysed sample which gave rise to the suggested genotype. The presence of a match may for part of the evidence against a suspect. The presence of a match may assist the direction and/or conduct of further enquiries, for instance by law enforcement authorities.

The sample may be taken from an individual and/or a location and/or an item.

The suggested genotype may be proposed manually, for instance by an operator. The suggested genotype may be suggested automatically. The suggested genotype may be suggested by an expert system. The suggested genotype may be suggested by an automated system.

The suggested genotype may be the only genotype considered by the method.

The adjustment may take the first stage profile, potentially through one or more intervening profiles, to the simulation profile. Preferably the simulation profile is an anticipation of the data profile which would be expected to occur for that genotype in practice. Preferably the adjustment simulates the experimentally determined profile expected for a suggested genotype. Preferably the adjustment accounts for a plurality of factors. The adjustment may be made in a single step or the adjustment for separate factors may be made in a series of separate steps.

The one or more factors may include one or more of preferential amplification and/or stutter and/or allele drop out and/or allele drop in and/or stochastic components and/or noise and/or the relative contributions from the sources.

The adjustment may alter the peak height and/or peak area and/or peak position and/or peak distribution for one or more of the allele identities. The adjustment may be based on a model of the factor's impact, particularly its impact on the peak height and/or peak area and/or peak position and/or peak distribution. One or more of the models may be theoretically determined. Preferably one or more of the models are derived from experimental evidence, such as prior investigations of the factor's impact. The model may be different for each factor. The model may be a distribution from which an adjustment term is taken at random. The distribution may be a normal distribution. The model may be a shaped distribution, particularly defined be beta parameters.

The adjustment for one or more of the separate factors preferably includes within the adjustment an account of the variable effect of the factor on different occasions. The adjustment for one or more of the separate factors preferably includes within the adjustment an account of the random nature of the extent of the effect of the factor on different occasions. Preferably the adjustment for the one or more separate factors does not apply an adjustment which has a fixed value for each occasion.

Particularly for preferential amplification a normal distribution is preferred as the model. In the case of preferential amplification preferably the normal distribution is used to generate number z, where z is the random number generated for the peak area of the lighter peak in a given contributor's genotype. Preferably the mean $\mu$ and standard deviation for the normal distribution at one or more and preferably each loci is estimated by computing a value $z_j$ for the $j^{th}$ heterozygote in the sample, the value $z_j$ representing the proportion of the total normalised peak area corresponding to the lighter of the heterozygote peaks.

Particularly for allele drop out, a zero peak height is applied to one or more of the peaks. Allele drop out can be modelled using the cumulative distribution function of a Normal distribution.

Particularly for stutter, a shaped distribution, particularly defined by a Beta parameter, is preferred as the model. In the case of stutter preferably the shaped distribution is defined by Beta parameters, where a and b are inferred from prior experimentally determined information. Preferably the mean and standard deviation for the prior information are deemed to give the shape parameters, a and b, by the equations :-Mean = a / (a+b) and Variance = (standard deviation)$^2$ = ab / [(a+b+1)(a+b)$^2$].

Particularly for noise, a shaped distribution, particularly defined by a Gamma distribution, is preferred as the model. The Gamma distribution may be defined, ideally at each allele position, by $Ga(\gamma, \delta)$, where $\gamma$ is the shape parameter and $\delta$ is the scale parameter. Preferably $\gamma$ and $\delta$ are determined from prior experimentally determined information. The model may assume noise acts independently at each of the peak positions. The model may assume that noise acts in an additive manner to the peak areas. The model may assume that noise depends on the total peak area observed in the data profile.

Preferably the accounting for one or more of the above factors adjusts the first stage profile to a modelled profile.

The comparing of the data profile and the simulation profile may be a statistically based measure of the level of match or correspondence between the data profile and the simulation profile.

The indication may be a likelihood ratio and/or a probability. The indication may include an assessment of the function $\int p(d \mid A_i, w, H_p) \, p(w) dw$ and/or $\int p(d \mid A_i, w, H) \, p(w) dw$.

Preferably the determination of the data profile and/or the suggestion of the suggested genotype and/or the provision of the first stage profile and/or the adjustment to the modelled profile and/or the adjustment to the simulation profile and/or the comparison and/or the indication are performed by an expert system and ideally by an automated process. Preferably all of the steps are provided by an expert system and ideally by an automated process. Preferably no expert input is required during application of the method to a sample.

Various embodiments of the invention will now be described, by way of example only, and with reference to the accompanying drawings in which:-

Figure 1 is an illustration of a measured profile at a particular locus;

Figure 2 is an illustration of a predicted pure profile for the locus of Figure 1 given assumed allele identities for the contributors;

Figure 3 is the profile of Figure 2 adjusted according to a model which counts for preferential amplification;

Figure 4 is the profile of Figure 3 still further adjusted according to a model allowing for stutter;

Figure 5 is the profile of Figure 4 still further adjusted to model a particular relative contribution from the first and second sources;

Figure 6 illustrates model information relating to preferential amplification for the various loci; and

Figure 7 illustrates model information relating to stutter for the various loci.

Forensic investigation of DNA is widely used to link or disprove a link between an individual and a DNA sample, and hence to a location, item or criminal act.

In many cases, the profile obtained by PCR amplification of the DNA sample relates only to a single individual. Some benefits of the invention, in the context of single source samples are discussed later in this document. In a number of cases, however, particularly in the sexual crimes area, the DNA sample obtained contains DNA from more than one individual. As a consequence this mixture needs to be considered in a different way when determining whether or not an individual contributed to the DNA in that sample. The number of samples classed as mixed also increases as the sensitivity of profiling techniques increases.

Presently experienced forensic scientists consider the profile results obtained from analysis of DNA samples and consider whether or not the mixture result would have been likely to have occurred given additional information on the potential contributors to the sample. In many cases one of the contributors to the sample will be known in terms of the victim and their DNA profile. The consideration may be, therefore, whether the other contribution or contributions to the sample came from a particular suspect or not. At present the expert considers the results and expresses an opinion as to whether or not the scenario could have given rise to the profile results.

Whilst such consideration is performed to a very high standard, consideration by an expert is inherently subjective. As a consequence extensive training is necessary to be able to prepare such opinions and they can involve significant effort in defending them in court.

As with all DNA analysis, it is desirable to be able to reduce the level of operator input required in making a determination, and ideally to render mixtures analysis suitable for investigation by expert systems and particularly through the use of automated systems. The present invention aims to achieve this by its consideration of DNA mixtures and as to how those might have arisen.

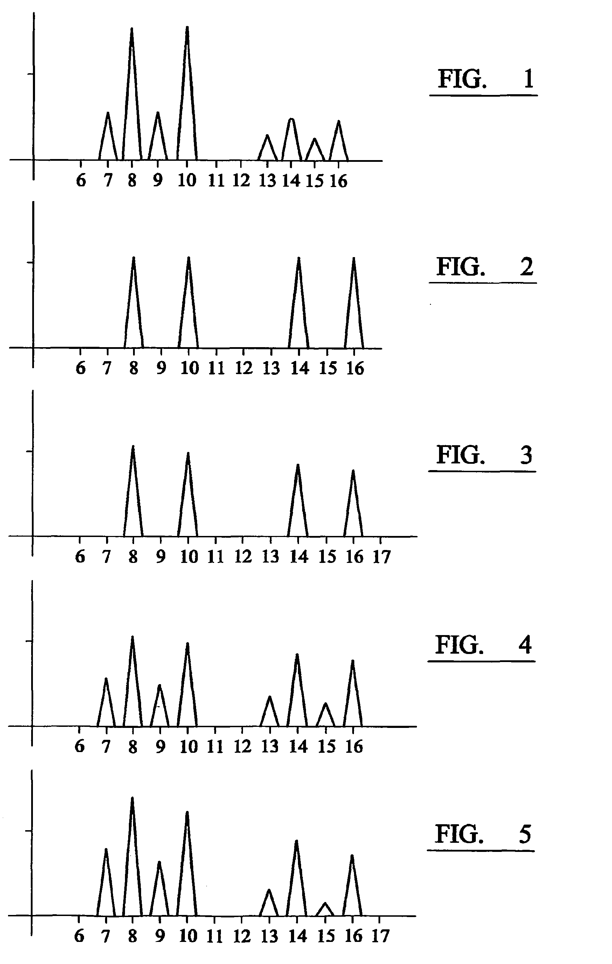

As illustrated in Figure 1, a DNA data profile for a sample at a particular locus has been obtained. The results indicate a varying strength of signal with different allele identity at the locus. The results strongly suggest, however, allele contribution at more than two of the alleles for that locus, and as a consequence the presence of a sample arising from a number of DNA sources.

The technique of the present invention aims to start with possible combinations of alleles, a suggested genotype, and then work towards the type of results which would actually be, a simulation profile, to see if it is practical that those possible combinations could have given rise to the actually measured data profile, as exemplified in Figure 1.

This approach stems from the following analysis of the position and the assessment which arises and needs solving as a result.

In general, both the prosecution and defence will advance hypotheses in terms of specific sets of contributing alleles to a scenario. The sets may be different or overlap partially or even wholly.

Thus, for the prosecution, $H_p$ : specifies a number of possible allele vectors $\underline{A}_1$.............$\underline{A}_m$ and for the defence, $H_d$ : specifies a potentially different set $\underline{B}_1$.............$\underline{B}_n$

The vector of peak areas is denoted $\underline{d}$. The vector of mixture proportions for the contributors is denoted w.

$$\text{The likelihood ratio } LR = \int LR(w)\, p(w)\, dw \;=\; \int \frac{\sum_{i=1}^{m} p(d, A_i \mid w, H_p)}{\sum_{i=1}^{n} p(d, B_i, \mid w, H_d)}\, p(w)\, dw$$

Factorising the joint densities $p(A_i, d \mid w \mid H_p)$ and $p(B_i, d \mid w \mid H_d)$ in a way that makes the likelihood ratio easy to evaluate we get $p(A_i, d \mid w \mid H_p) = p(d \Rightarrow A_i, w, H_p)\, Pr(A_i \Rightarrow w, H_p)$ and $p(B_i, d \mid w \mid H_d) = p(d \Rightarrow B_i, w, H_d)\, Pr(B_i \Rightarrow w, H_d)$.

$$LR = \int \frac{\sum_{i=1}^{m} p(d \mid A_i, w, H_p) \, Pr(A_i \mid w, H_p)}{\sum_{i=1}^{n} p(d \mid B_i, w, H_d) \, Pr(B_i \mid w, H_d)} \; p(w)dw$$

Assuming that $Pr(A_i \Rightarrow w, H_p) = Pr(A_i \Rightarrow H_p)$ and $Pr(B_i \Rightarrow w, H_d) = Pr(B_i \Rightarrow H_d)$, i.e. assuming that the probability of observing genotype $A_i$ in the population is independent of its strength in the mixture we obtain

$$LR = \int \frac{\sum_{i=1}^{m} p(d \mid A_i, w, H_p) \, Pr(A_i \mid H_p)}{\sum_{i=1}^{n} \int p(d \mid B_i, w, H_d) \, Pr(B_i \mid H_d)} \; p(w)dw$$

This equation contains the repeated assessment of a sum of the type

$$\sum_i p(d \Rightarrow A_i, w, H) \, Pr(A_i \Rightarrow H)$$

The $Pr(A_i \Rightarrow H)$ are genotype probabilities which can be calculated easily and so we focus on the assessment of $p(d \Rightarrow A_i, w, H)$ which becomes the critical term requiring assessment in the subsequent routes.

As a way of progressing the assessment the technique seeks to compare the data profile actually measured with a simulation profile. The data profile is generally already available in graphical form from the measurement process and it can be considered in that form or in a subsequent conversion into numerical terms. The generation of the simulation profile is now discussed and again this can be progressed graphically or in the underlying numerical form.

The start of the simulation process is illustrated with regard to Figure 2 in which one such, hypothetical first stage profile is illustrated. This first stage profile is generated from a suggested genotype. The profile is in effect a representation of the result expected with particular alleles contributing to the sample and assuming equivalence in the relative proportions of the sample arising from each of the two sources. The profile is a pure profile, however, and does not reflect any of the other factors contributing to an actual data profile as would arise from measurements. In this case allele identities 8, 10 are assumed to arise from one individual and alleles 14 and 16 from a second individual. The profile outside of those allele identities reflects the pure measurement and gives a zero result.

This first stage profile is then adjusted according to a model which aims to represent preferential amplification effects. Preferential amplification refers to the tendency of lower molecular weight alleles to amplify to a higher level than higher weight alleles in a sample given equivalence in the amount of each allele originally present. As a consequence the second stage profile resulting in Figure 3 indicates the distorting effect of this preferential amplification on the second stage profile arising, 8, 10, 14 and 16.

Allele drop out can be adjusted for as a separate adjustment or even as an extreme treatment of preferential amplification in which one allele pair is treated as having zero height/area.

The method also seeks to account for and adjust for the effect of other variables which could give rise to discrepancies between the actual allele identity and the data profile. Thus in Figure 4 the effect of stutter is accounted for. Stutter basically gives rise to an increased peak area at the n-4 position and a decrease at the n position accordingly. As a result, the profiles 8, 10, 14, 16 are skewed slightly to the left, the third stage profile, compared with the Figure 3 form, and peaks at alleles 7, 9, 13 and 15 occur.

In a similar way, effects such as allele drop out, allele drop in, noise and other artefact effects can be accounted for in further stage profiles. Other effects, such as stochastic components, can also be accounted for in a similar manner in still further stage profiles.

The order in which the adjustments are made is not significant.

Once all of these effects have been accounted for a modelled profile is reached.

It is also necessary to consider the fact that the sources may not have contributed equally to the sample. As a consequence a further adjustment to the modelled profile is necessary to get the simulation profile. This adjustment is illustrated in relation to Figure 5 where for this simulation it is assumed that two thirds of the DNA is contributed by the individual behind alleles 8 and 10, and one third by the individual behind alleles 14 and 15. As a consequence the profile is higher in respect of allele identities 8 and 10 than in respect of 14 and 15 in the respective proportions. Other relative contributions can be reflected in other adjustments. The overall result is to convert the modelled profile into a simulation profile.

The overall aim is to convert the allele identities used as the first stage profile in Figure 2 into a simulation profile, Figure 5, which would be expected to occur in a real world measurement according to the simulation process. This simulation profile can then be compared with the data profile that was actually measured, for instance Figure 1, to see how effective a simulation has occurred.

The models used to take the first stage profile through to the modelled profile can be theoretically based or based on previous experimentation. For instance, examination of the data from several hundred heterozygotes allows the generation of a model in respect of each of the key variations.

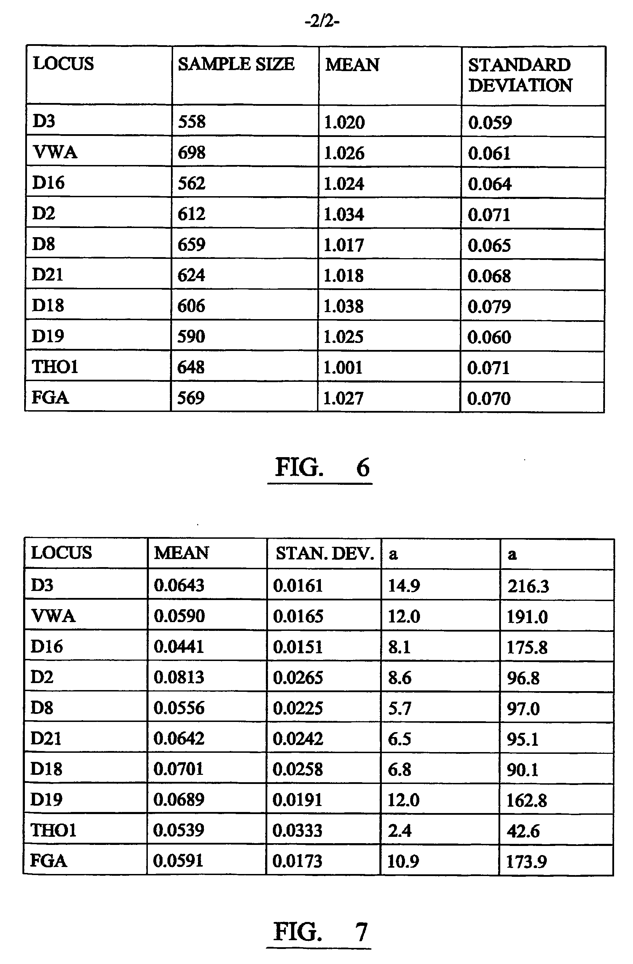

In the case of preferential amplification the applicant's internal data supports the allocation of a model having a normal distribution to generate number z, where z is the random number generated for the peak area of the lighter peak in a given contributor's genotype. A constrained normal distribution is preferred. The mean, u and standard deviation for the normal distribution at each loci is estimated in the following way. For the $j^{th}$ heterozygote in the sample, a value $z_j$ was computed, which represents the proportion of the total normalised peak area corresponding to the lighter of the heterozygote peaks. Figure 6 shows in tabular form the mean and standard deviation of these z values calculated for each of the preferred loci for the analysis. The figures obtained correspond to the standard maximum likelihood estimates.

In the case of stutter internal records of the applicant where considered and a shaped distribution deemed most appropriate. Stutter was assumed to apply to peaks independently of one another. The Beta parameters, a and b, were inferred from the prior information. The mean and standard deviation for the prior information are illustrated in Figure 7 and these lead to the shape parameters, a and b, by the following equations :-

$$\text{Mean} = a / (a+b)$$

$$\text{Variance} = (\text{standard deviation})^2 = ab / [(a+b+1)(a+b)^2]$$

In the case of noise the internal records of the applicant indicate that a Gamma distribution is the most appropriate. The effect of noise is modelled as a function of the total peak area. The effect of nosie is assumed to apply independently of allele/peak position. The Gamma distribution is defined by $Ga(\gamma,\delta)$, at each allele positions, where $\gamma$ is the shape parameter and $\delta$ is the scaling parameter. The values of $\gamma$ and $\delta$ are determined from prior experimentation. The effect of noise is assumed to be additive.

As well as the above mentioned factors it is possible to adjust for the effect of preferential degradation when needed. In this context reference is made to the applicant's technique set out in UK Patent Application No 0130675.2, filed 21 December 2001 under reference P17961 and continued on the same date as this PCT patent application, also as a PCT patent application, bearing reference P17961WO. The contents of that document are incorporated herein by reference, particularly as regards the details of the way in which the effect of preferential degradation varies across loci and hence the manner in which it may be accounted for. A relationship of the type derived by the techniques set out in those documents can be used to adjust a profile towards the simulation profile and so account in that process for preferential degradation effects.

Once a simulation profile has been generated in this way the extent to which it matches the actual measured profile can be established. A variety of statistical tools can be used to measure the degree of match.

By way of example it is possible to use Monte Carlo based comparisons in which the simulation profile and data profile each define a data vector and the relative separation of these can be quantified. The comparison of any simulation profile data vector and data profile data vector gives rise to a distance separation, possibly expressed in Euclidean distance. Better matches may be deemed to be those within a distance Q of the measured data vector; the number of matches, x, out of the total set of attempts, N, may be used as a proxy for the probability density function. This is possible because the proxy is constructed to be from terms in the numerator and denominator. The proxy used is $\text{Proxy}_1 = x/N$. The proxy serves to eliminate many of the solutions and hence renders the number of relevant solutions processable in a reasonable time frame. A proxy is calculated across the set of loci being considered. The proxy probability values give informative unbiased information.

As most profiling techniques consider a large number of loci to give statistically significant results this means a great deal of scenarios which must be considered. For example the generally used profile in the UK involves 11 loci. These 11 loci must be considered at each of a large number of relative contribution levels (weightings) and each of those combinations must be considered together with a vast number of possible genotypes. A massive number of simulations and calculations thus result and for which the degree of match needs to be considered. To render such situations processable in practical circumstances alternative techniques can be used.

Foremost amongst the possibilities is the use of Markov Chain Monte Carlo analysis; a special case amongst the general techniques of Monte Carlo. Within this class of methods a variety of possibilities exist including Gibbs samplers, continuous time algorithms and dimension jumping methods.

In basic terms this technique is used because of its ability to approximate complex mathematical integrals and/or very large summations. This might occur in either or both of the numerator and denominator of the likelihood ratios expressed above.

The space defined by all of the possible points for which the calculations can be performed is sampled by starting at a first point. For that point a comparison between the data profile and the simulation profile is performed to give an expression of the match; a posterior density. It is preferred that the first point is one with a high density, but not essential. From that point a move on through space is suggested and the process is repeated at that point. If the move is to a poor point in terms of its probability then an alternative point may be suggested. If the probability is high at a point then a large number of points close by are likely to be considered to map out such hotspots. If the probability is low then wide space points are used to cross this space until another hotspot is encountered. The idea is to sufficiently fully sample the space to pick up all the high probability points whilst avoiding the vast majority of the points in the space which are low probability points.

As well as offering benefits in the context of samples which are mixtures, due to their having been contributed to by more than one person, the invention also offers benefits in the context of single source samples, or those samples which are believed to be single source samples.

When a sample is collected and profiled experimentally, it is useful to be able to enter a record of the genotype believed to be behind that profile into a database. That genotype can then be searched against existing genotype records and/or itself be searched against in the future. Such searches can generate an indication of a match between the genotypes derived from separate samples and hence imply a link between those samples. A key feature of this process is that the genotype determined to be behind a profile and sample result is correctly determined. At present this process generally involves taking the experimental profile and applying a series of rules to it to reduce it to the underlying genotype.

The present invention offers an alternative way of achieving this determination and enables the genotype determined to be fully validated.

In this context, the method involves collecting and experimentally determining a profile for the sample as before. However, rather than adjust the experimental, data profile, to get to the underlying genotype, the technique of the invention generates one or more suggested genotypes. For each suggested genotype the simulations approach discussed above is applied. Thus the suggested genotype is used to determine a first stage profile and this is then adjusted to account for one or more of the factors discussed above. Thus preferential amplification, stutter, noise etc can be accounted for. A simulation profile results and it is then possible to compare that with the data profile and so provide an indication as to the likelihood of the data profile given the suggested genotype. In the context of true single source samples, the indication of likelihood would be expected to be strong. Furthermore, a strong indication in respect of only one suggested genotype would be expected, with other suggested genotypes giving poorer indications of likelihood. However, the technique would also provide useful indications that the sample was in fact a mixture and needed to be considered as such, in cases where the prior art approaches would deem the sample to be a true single source sample.

The validated suggested genotype can be recorded on a database and/or used in any other way that a genotype is presently considered. Searches for matches with such a genotype are thus possible. The validation provides greater confidence, however, that the genotype entered on the database or otherwise used is correct and no an incorrect interpretation of the experimental data profile.

**Claims**

1. A method for providing an indication of the likelihood of a data profile being given by a suggested genotype for a DNA sample, the method including:

   a) analysing the sample to produce a data profile for the sample for a locus, the data profile including a peak height and/or peak area at one or more allele identities;
   b) proposing a suggested genotype;
   the method being **characterised in that** the method also includes:
   c) generating a first stage profile for the locus for the suggested genotype, the first stage profile including a peak height and/or peak area at each allele identity in the suggested genotype;
   d) adjusting the first stage profile to account for one or more factors to give a simulation profile, the one or more factors including at least one of preferential amplification and/or stutter and/or allele dropout and/or allele drop in and/or stochastic components and/or noise, the adjustment altering the peak height and/or peak areas and/or peak position and/or peak distribution for one or more of the allele identities;

e) comparing the data profile and the simulation profile to provide an indication of the likelihood of the data profile being given by the suggested genotype, the comparing of the data profile and the simulation profile being a statistically based measure of the level of match or correspondence between the data profile and the simulation profile;

and wherein, the generation of the first stage profile and the adjustments to the first stage profile and the comparison of the data profile and the simulation method are performed by an expert system or by an automated process.

2. A method according to claim 1 in which the method is repeated for a plurality of loci and/or for a plurality of proposed genotypes.

3. A method according to claim 1, the method including analysing the sample to produce a data profile for the sample for a plurality of loci;

proposing a plurality of suggested genotypes for the plurality of sources; generating a first stage profile for the locus for one of the suggested genotypes; adjusting the first stage profile to account for one or more factors to give a simulation profile for the one of the suggested genotypes;

comparing the data profile and the simulation profile for the one of the suggested genotypes to provide an indication of the likelihood of the data profile given the one suggested genotype;

repeating these stages for each of the other suggested genotypes.

4. A method according to claim 1, for investigating the potential sources of a DNA sample arising from a plurality of sources, the method including:-

analysing the sample to produce a data profile for the sample for a locus;

proposing a suggested genotype for the plurality of sources;

generating a first stage profile for the locus for the suggested genotype; adjusting the first stage profile to account for one or more factors to give a simulation profile;

comparing the data profile and the simulation profile to provide an indication of the likelihood of the data profile given the suggested genotype.

5. A method according to claim 4 in which the method is repeated for a plurality of loci and/or for a plurality of proposed genotypes.

6. A method according to claim 3, for providing information on the likelihood of the results of the analysis of a DNA sample given one or more particular genotypes, the DNA sample arising from a plurality of sources, the method including:-

analysing the sample to produce a data profile for the sample for a plurality of loci;

proposing a plurality of suggested genotypes for the plurality of sources;

generating a first stage profile for the locus for one of the suggested genotypes;

adjusting the first stage profile to account for one or more factors to give a simulation profile for the one of the suggested genotypes;

comparing the data profile and the simulation profile for the one of the suggested genotypes to provide an indication of the likelihood of the data profile given the one suggested genotype;

repeating these stages for each of the other suggested genotypes.

7. A method according to any preceding claim in which the indication is a possible or not possible indication for that data profile given a particular suggested genotype.

8. A method according to any of claims 1 to 6 in which the indication is a likelihood ratio relating the likelihood of the data profile given one or more first suggested genotypes to the likelihood of the data profile given one or more second suggested genotypes.

9. A method according to any preceding claim in which the suggested genotype is proposed manually, for instance by an operator.

10. A method according to any preceding claim in which the suggested genotype is suggested automatically.

11. A method according to any preceding claim in which the adjustment takes the first stage profile, potentially through one or more intervening profiles, to the simulation profile, the simulation profile being an anticipation of the data profile which would be expected to occur for that suggested genotype in practice.

12. A method according to any preceding claim in which the adjustment simulates the experimentally determined profile expected for a suggested genotype.

13. A method according to any preceding claim in which the adjustment alters the peak height and/or peak area and/or peak position and/or peak distribution for one or more of the allele identities.

14. A method according to any preceding claim in which the adjustment is based on a model of the factor's impact, particularly its impact on the peak height and/or peak area and/or peak position and/or peak distribution.

15. A method according to claim 14 in which one or more of the models are theoretically determined.

16. A method according to claim 14 in which one or more of the models are derived from experimental evidence, such as prior investigations of the factor's impact.

17. A method according to any preceding claim in which the adjustment for one or more of the separate factors includes within the adjustment an account of the variable effect of the factor on different occasions.

18. A method according to any preceding claim in which the adjustment for one or more of the separate factors includes within the adjustment an account of the random nature of the extent of the effect of the factor on different occasions.

19. A method according to any preceding claim in which the first stage profile and/or modelled profile is adjusted to give the simulation profile by making an adjustment to account for the relative contributions of the two or more sources to the sample.

20. A method according to any preceding claim in which the indication of the likelihood of the data profile given the suggested genotype is used to propose one or more search genotypes from amongst the suggested genotypes, the one or more search genotypes being those suggested genotypes for which the indication of the likelihood of the data profile given that suggested genotype is the highest or amongst the highest.

21. A method according to claim 20 in which the one or more search genotypes are recorded or otherwise entered into a database.

22. A method according to claim 20 or claim 21 in which the one or more search genotypes are searched against the contents of a database, the search being for one or more database genotypes that match the one or more search genotypes.

23. A method according to 22 in which the presence of a match between a search genotype and a database genotype is used as an indication of a link between the search genotype and the database genotype, for instance that source of the search genotype and the database genotype were the same person.

24. A method according to any of claims 20 to 23 in which the one or more search genotypes are recorded or otherwise entered into the database as part of the searching process and the one or more search genotypes can themselves be searched against as database genotypes in future searches.

25. A method according to any of claims 20 to 23 in which the one or more search genotypes are subjected to the searching process, without being recorded or otherwise entered into the database.

26. A method according to any preceding claim in which the indication is a likelihood ratio and/or a probability.

27. A method according to any preceding claim in which the indication includes an assessment of the function $p(d \mid A_i, w, H)$.

28. A method according to any preceding claim in which the determination of the data profile and/or the suggestion of the suggested genotype and/or the provision of the first stage profile and/or the adjustment to the modelled profile and/or the adjustment to the simulation profile and/or the comparison and/or the indication are performed by an expert system and ideally by an automated process.

29. A method according to any preceding claim in which the indication of the likelihood of the data profile given the suggested genotype is preferably used to determine the genotype assigned to that sample, for instance for future search and other consideration purposes.

30. A method according to any preceding claim in which, where the indication of likelihood meets predetermined criteria that suggested genotype is accepted as representing that sample.

**Patentansprüche**

1. Verfahren zur Bereitstellung einer Angabe der Wahrscheinlichkeit, dass ein Datenprofil durch einen vorgeschlagenen Genotyp für eine DNA-Probe gegeben ist, wobei das Verfahren einschließt:

   a) Analyse der Probe, um ein Datenprofil für die Probe für einen Locus zu erzeugen, wobei das Datenprofil eine Peak-Höhe und/oder Peak-Fläche bei einer oder mehreren Aliel-Identitäten einschließt;
   b) Vorschlagen eines vorgeschlagenen Genotyps;
   wobei das Verfahren **dadurch gekennzeichnet ist, dass** das Verfahren ebenfalls einschließt:
   c) Erzeugen eines Anfangsphasen-Profils für den Locus für den vorgeschlagenen Genotyp, wobei das Anfangsphasen-Profil eine Peak-Höhe und/oder Peak-Fläche bei jeder Allel-Identität in dem vorgeschlagenen Genotyp einschließt;
   d) Anpassen des Anfangsphasen-Profils, um einem oder mehreren Faktoren Rechnung zu tragen, um ein Simulationsprofil zu ergeben, wobei der eine oder die mehreren Faktoren mindestens eines aus einer bevorzugten Amplifikation und/oder von Stottern und/oder von Allel-Wegfall und/oder von unerwartetem Allel-Auftreten und/oder von stochastischen Komponenten und/oder von Hintergrundrauschen einschießen, wobei die Anpassung die Peak-Höhe und/oder Peak-Flächen und/oder Peak-Position und/oder Peak-Verteilung für eine oder mehrere der Allel-Identitäten verändert;
   e) Vergleichen des Datenprofils und des Simulationsprofils, um eine Angabe der Wahrscheinlichkeit bereitzustellen, dass das Datenprofil durch den vorgeschlagenen Genotyp gegeben ist, wobei der Vergleich des Datenprofils und des Simulationsprofils eine auf Statistik beruhende Messung des Übereinstimmungsmaßes oder der Korrespondenz zwischen dem Datenprofil und dem Simulationsprofil ist;
   und wobei die Erzeugung des Anfangsphasen-Profils und der Anpassung des Anfangsphasen-Profils und der Vergleich des Datenprofils und des Simulationsverfahrens durch ein Expertensystem oder durch ein automatisiertes Verfahren durchgeführt werden.

2. Verfahren nach Anspruch 1, wobei das Verfahren für eine Mehrzahl von Loci und/oder für eine Mehrzahl von vorgeschlagenen Genotypen wiederholt wird.

3. Verfahren nach Anspruch 1, wobei das Verfahren einschließt:

   Analysieren der Probe, um ein Datenprofil für die Probe für eine Mehrzahl von Loci zu erzeugen;
   Vorschlagen einer Mehrzahl von vorgeschlagenen Genotypen für die Mehrzahl von Quellen;
   Erzeugen eines Anfangsphasen-Profils für den Locus für einen der vorgeschlagenen Genotypen;
   Anpassen des Anfangsphasen-Profils, um einem oder mehreren Faktoren Rechnung zu tragen, um ein Simulationsprofil für den einen der vorgeschlagenen Genotypen zu ergeben;
   Vergleichen des Datenprofils und des Simulationsprofils für den einen der vorgeschlagenen Genotypen, um eine Angabe der Wahrscheinlichkeit bereitzustellen, dass das gegebene Datenprofil durch den einen vorgeschlagenen Genotyp gegeben ist;
   Wiederholung dieser Stufen für jeden der anderen vorgeschlagenen Genotypen.

4. Verfahren nach Anspruch 1 zum Untersuchen der potenziellen Quellen einer DNA-Probe, die aus einer Mehrzahl von Quellen stammt, wobei das Verfahren einschließt:

   Analysieren der Probe, um ein Datenprofil für die Probe für einen Locus zu erzeugen;
   Vorschlagen eines vorgeschlagenen Genotyps für die Mehrzahl von Quellen;
   Erzeugen eines Anfangsphasen-Profils für den Locus für den vorgeschlagenen Genotyp;

Anpassen des Anfangsphasen-Profils, um einem oder mehreren Faktoren Rechnung zu tragen, um ein Simulationsprofil zu ergeben;

Vergleichen des Datenprofils und des Simulationsprofils, um eine Angabe für die Wahrscheinlichkeit bereitzustellen, dass das Datenprofil durch den vorgeschlagenen Genotyp gegeben ist.

5. Verfahren nach Anspruch 4, wobei das Verfahren für eine Mehrzahl von Loci und/oder für eine Mehrzahl von vorgeschlagenen Genotypen wiederholt wird.

6. Verfahren nach Anspruch 3 zur Bereitstellung von Information über die Wahrscheinlichkeit, dass die Ergebnisse der Analyse einer DNA-Probe durch einen oder mehrere spezielle Genotypen gegeben ist, wobei die DNA-Probe aus einer Mehrzahl von Quellen stammt, wobei das Verfahren einschließt:

Analysieren der Probe, um ein Datenprofil für die Probe für eine Mehrzahl von Loci zu erzeugen;

Vorschlagen einer Mehrzahl von vorgeschlagenen Genotypen für die Mehrzahl von Quellen;

Erzeugen eines Anfangsphasen-Profils für den Locus für einen der vorgeschlagenen Genotypen;

Anpassen des Anfangsphasen-Profils, um einem oder mehreren Faktoren Rechnung zu tragen, um ein Simulationsprofil für den einen der vorgeschlagenen Genotypen zu ergeben;

Vergleichen des Datenprofils und des Simulationsprofils für den einen der vorgeschlagenen Genotypen, um eine Angabe der Wahrscheinlichkeit bereitzustellen, dass das Datenprofil durch den einen vorgeschlagenen Genotyp gegeben ist;

Wiederholen dieser Stufen für jeden der anderen vorgeschlagenen Genotypen.

7. Verfahren nach irgendeinem der vorangehenden Ansprüche, wobei die Angabe eine Angabe "möglich oder nicht möglich" dafür ist, dass das Datenprofil einen speziellen vorgeschlagenen Genotyp ergibt.

8. Verfahren nach irgendeinem der Ansprüche 1 bis 6, wobei die Angabe ein Wahrscheinlichkeitsverhältnis ist, welches die Wahrscheinlichkeit, dass das Datenprofil durch einen oder mehrere erste nahgelegte Genotypen gegeben ist, mit der Wahrscheinlichkeit, dass das Datenprofil durch einen oder mehrere zweite vorgeschlagene Genotypen gegeben ist, in Beziehung setzt.

9. Verfahren nach irgendeinem vorangehenden Anspruch, wobei der vorgeschlagene Genotyp manuell, zum Beispiel durch eine Bedienungsperson, vorgeschlagen wird.

10. Verfahren nach irgendeinem vorangehenden Anspruch, wobei der vorgeschlagene Genotyp automatisch vorgeschlagen wird.

11. Verfahren nach irgendeinem vorangehenden Anspruch, wobei die Anpassung das Anfangsphasen-Profil, potenziell durch ein oder mehrere Zwischen-Profile, zu dem Simulationsprofil geführt wird, wobei das Simulationsprofil eine Vorwegnahme des Datenprofils ist, für welches erwartet würde, dass es für den vorgeschlagenen Genotyp in der Praxis vorkommt.

12. Verfahren nach irgendeinem vorangehenden Anspruch, wobei die Anpassung das experimentell bestimmte Profil simuliert, das für einen vorgeschlagenen Genotyp erwartet wird.

13. Verfahren nach irgendeinem vorangehenden Anspruch, wobei die Anpassung die Peak-Höhe und/oder Peak-Fläche und/oder Peak-Position und/oder Peak-Verteilung für eine oder mehrere der Allel-Identitäten ändert.

14. Verfahren nach irgendeinem vorangehenden Anspruch, wobei die Anpassung auf einem Modell des Einflusses des Faktors beruht, insbesondere seines Einflusses auf die Peak-Höhe und/oder Peak-Fläche und/oder Peak-Position und/oder Peak-Verteilung.

15. Verfahren nach Anspruch 14, wobei eines oder mehrere der Modelle theoretisch bestimmt werden.

16. Verfahren nach Anspruch 14, wobei eines oder mehrere der Modelle aus experimentellen Fakten abgeleitet sind, wie früheren Untersuchungen des Einflusses des Faktors.

17. Verfahren nach irgendeinem vorangehenden Anspruch, wobei die Anpassung für einen oder mehrere der getrennten Faktoren innerhalb der Anpassung eine Berücksichtigung des variablen Effekts des Faktors bei verschiedenen Anlässen einschließt.

**18.** Verfahren nach irgendeinem vorangehenden Anspruch, wobei die Anpassung für einen oder mehrere der getrennten Faktoren innerhalb der Anpassung eine Berücksichtigung der statistischen Natur des Ausmaßes der Auswirkung des Faktors bei verschiedene Gelegenheiten einschließt.

**19.** Verfahren nach irgendeinem vorangehenden Anspruch, wobei das Anfangsphasen-Profil und/oder modellierte Profil angepasst wird, um das Simulationsprofil zu ergeben, in dem eine Anpassung gemacht wird, um den relativen Beiträgen der zwei oder mehr Quellen zu der Probe Rechnung zu tragen.

**20.** Verfahren nach irgendeinem vorangehenden Anspruch, wobei die Angabe der Wahrscheinlichkeit, dass das Datenprofil durch den vorgeschlagenen Genotyp gegeben ist, verwendet wird, um einen oder mehrere Such-Genotypen aus den vorgeschlagenen Genotypen vorzuschlagen, wobei der eine oder die mehreren Such-Genotypen jene vorgeschlagenen Genotypen sind, bei denen die Angabe der Wahrscheinlichkeit, dass das Datenprofil durch den vorgeschlagenen Genotyp gegeben ist, die höchste oder unter den höchsten ist.

**21.** Verfahren nach Anspruch 20, wobei der eine oder die mehreren Such-Genotypen aufgezeichnet oder auf andere Weise in eine Datenbank eingegeben werden.

**22.** Verfahren nach Anspruch 20 oder Anspruch 21, wobei der eine oder die mehreren Such-Genotypen im Inhalt einer Datenbank gesucht werden, wobei die Suche nach einem oder mehrere Datenbank-Genotypen ist, die mit dem einen oder den mehreren Such-Genotypen übereinstimmen.

**23.** Verfahren nach Anspruch 22, wobei die Anwesenheit einer Übereinstimmung zwischen einem Such-Genotyp und einem Datenbank-Genotyp als Angabe einer Verknüpfung zwischen dem Such-Genotyp und dem Datenbank-Genotyp verwendet wird, zum Beispiel, dass die Quelle des Such-Genotyps und des Datenbank-Genotyps dieselbe Person waren.

**24.** Verfahren nach irgendeinem der Ansprüche 20 bis 23, wobei der eine oder die mehreren Such-Genotypen als Teil des Suchverfahrens aufgezeichnet oder auf andere Weise in die Datenbank eingegeben werden und der eine oder die mehreren Such-Genotypen selbst als Datenbank-Genotypen bei zuki7nftigen Suchen gesucht werden können.

**25.** Verfahren nach irgendeinem der Ansprüche 20 bis 23, wobei der eine oder die mehreren Such-Genotypen dem Suchverfahren unterzogen werden, ohne dass sie aufgezeichnet oder auf andere Weise in die Datenbank eingegeben werden.

**26.** Verfahren nach irgendeinem der vorangehenden Ansprüche, wobei die Angabe ein Wahrscheinlichkeitsverhältnis und/oder eine Wahrscheinlichkeit ist.

**27.** Verfahren nach irgendeinem der vorangehenden Ansprüche, wobei die Angabe eine Abschätzung der Funktion $p(d \mid A_i, w, H)$ einschließt.

**28.** Verfahren nach irgendeinem der vorangehenden Ansprüche, wobei die Bestimmung des Datenprofils und/oder des Naheliegens des vorgeschlagenen Genotyps und/oder die Bereitstellung des Anfangsphasen-Profils und/oder die Anpassung an das modellierte Profil und/oder die Anpassung an das Simulationsprofil und/oder der Vergleich und/oder die Angabe durch ein Expertensystem und idealerweise durch ein automatisiertes Verfahren durchgeführt werden.

**29.** Verfahren nach irgendeinem vorangehenden Anspruch, wobei die Angabe der Wahrscheinlichkeit, dass das Datenprofil durch den vorgeschlagenen Genotyp gegeben ist, vorzugsweise verwendet wird, um den Genotyp, der dieser Probe zugeordnet wurde, zu bestimmen, zum Beispiel für eine zukünftige Suche und für die Zwecke anderer Überlegungen.

**30.** Verfahren nach irgendeinem vorangehenden Anspruch, wobei, wenn die Angabe der Wahrscheinlichkeit vorbestimmte Kriterien erfüllt, der vorgeschlagene Genotyp als eine Repräsentation der Probe akzeptiert wird.

**Revendications**

1. Procédé visant à fournir une indication de la vraisemblance d'un profil de données fourni par un génotype suggéré pour un échantillon d'ADN, le procédé comprenant :

   a) l'analyse de l'échantillon, afin de produire un profil de données pour l'échantillon pour un locus, le profil de données comprenant une hauteur de pic et/ou une superficie de pic au niveau d'une ou de plusieurs identités d'allèles ;
   b) la proposition d'un génotype suggéré ;
   le procédé étant **caractérisé en ce que** le procédé comprend également :
   c) la génération d'un premier profil de phase pour le locus pour le génotype suggéré, le premier profil de phase comprenant une hauteur de pic et/ou une superficie de pic à chaque identité d'allèle dans le génotype suggéré ;
   d) l'ajustement du premier profil de phase pour représenter un ou plusieurs facteurs visant à donner un profil de simulation, le ou les facteurs comprenant au moins un élément parmi l'amplification préférentielle et/ou l'hésitation et/ou la perte d'allèle et/ou la chute d'allèle et/ou les composants stochastiques et/ou le bruit, l'ajustement altérant la hauteur de pic et/ou les superficies de pic et/ou la position de pic et/ou la distribution de pic pour une ou plusieurs des identités d'allèle ;
   e) la comparaison du profil de données et du profil de simulation pour fournir une indication de la vraisemblance du profil de données fourni par le génotype suggéré, la comparaison du profil de données et du profil de simulation étant une mesure statistique du niveau d'adéquation ou de correspondance entre le profil de données et le profil de simulation ;
   et dans lequel la génération du premier profil de phase et les ajustements du premier profil de phase et la comparaison du profil de phase et du procédé de simulation sont effectués par un système expert ou par un processus automatisé.

2. Procédé selon la revendication 1, dans lequel le procédé est répété pour une pluralité de locus et/ou pour une pluralité de génotypes proposés.

3. Procédé selon la revendication 1, ledit procédé comprenant
   l'analyse de l'échantillon, afin de produire un profil de données pour l'échantillon, pour une pluralité de locus ;
   la proposition d'une pluralité de génotypes suggérés pour la pluralité de sources ;
   la génération d'un premier profil de phase pour le locus pour un ou plusieurs des génotypes suggérés ;
   l'ajustement du premier profil de phase pour représenter un ou plusieurs facteurs afin de donner un profil de simulation pour le génotype suggéré ;
   la comparaison du profil de données et du profil de simulation pour les génotypes suggérés, afin de fournir une indication de la vraisemblance du profil de données au vu du génotype suggéré ;
   la répétition de ces phases pour chacun des autres génotypes suggérés.

4. Procédé selon la revendication 1, visant à étudier les sources potentielles d'un échantillon d'ADN provenant d'une pluralité de sources, le procédé comprenant :

   l'analyse de l'échantillon, afin de produire un profil de données pour l'échantillon, pour un locus ;
   la proposition d'un génotype suggéré pour la pluralité de sources ;
   la génération d'un premier profil de phase pour le locus, pour le génotype suggéré ;
   l'ajustement du premier profil de phase, afin de représenter un ou
   plusieurs facteurs pour donner un profil de simulation ;
   la comparaison du profil de données et du profil de simulation, afin de fournir une indication de la vraisemblance du profil de données, au vu du génotype suggéré.

5. Procédé selon la revendication 4, dans lequel le procédé est répété pour une pluralité de locus et/ou pour une pluralité de génotypes proposés.

6. Procédé selon la revendication 3, visant à fournir des informations sur la vraisemblance des résultats de l'analyse d'un échantillon d'ADN, au vu d'un ou plusieurs génotypes particuliers, l'échantillon d'ADN provenant d'une pluralité de sources, le procédé comprenant :

   l'analyse de l'échantillon, afin de produire un profil de données pour l'échantillon, pour une pluralité de locus ;
   la proposition d'une pluralité de génotypes suggérés pour la pluralité de sources ;

la génération d'un premier profil de phase pour le locus pour un des génotypes suggérés ;

l'ajustement du premier profil de phase, pour représenter un ou plusieurs facteurs, afin de donner un profil de simulation pour l'un des génotypes suggérés ;

la comparaison du profil de données et du profil de simulation pour l'un des génotypes suggérés, afin de fournir une indication de la vraisemblance du profil de données, au vu du génotype suggérés :

la répétition de ces phases pour chacun des autres génotypes suggérés.

7. Procédé selon l'une quelconque des revendications précédentes, dans lequel l'indication est une indication possible ou non possible pour ce profil de données, au vu d'un génotype suggéré particulier.

8. Procédé selon l'une quelconque des revendications 1 à 6, dans lequel l'indication est un rapport de vraisemblance relatif à la vraisemblance du profil de données, au vu d'un ou plusieurs premiers génotypes suggérés, relativement à la vraisemblance du profil de données, au vu d'un ou plusieurs seconds génotypes suggérés.

9. Procédé selon l'une quelconque des revendications précédentes, dans lequel le génotype suggéré est proposé manuellement, par exemple par un opérateur.

10. Procédé selon l'une quelconque des revendications précédentes, dans lequel le génotype suggéré est suggéré automatiquement.

11. Procédé selon l'une quelconque des revendications précédentes, dans lequel l'ajustement prend le premier profil de phase, potentiellement par le biais d'un ou plusieurs profils d'intervention, au profil de simulation, le profil de simulation étant une anticipation du profil de données, qui serait supposé survenir pour ce génotype suggéré en pratique.

12. Procédé selon l'une quelconque des revendications précédentes, dans lequel l'ajustement simule le profil déterminé de manière expérimentale attendu pour un génotype suggéré.

13. Procédé selon l'une quelconque des revendications précédentes, dans lequel l'ajustement altère la hauteur de pic et/ou la superficie de pic et/ou la position de pic et/ou la distribution de pic pour une ou plusieurs des identités d'allèle.

14. Procédé selon l'une quelconque des revendications précédentes, dans lequel l'ajustement est basé sur un modèle de l'impact du facteur, en particulier son impact sur la hauteur de pic et/ou la superficie de pic et/ou la position de pic et/ou la distribution de pic.

15. Procédé selon la revendication 14, dans lequel un ou plusieurs des modèles sont théoriquement déterminés.

16. Procédé selon la revendication 14, dans lequel un ou plusieurs des modèles sont tirés d'une preuve expérimentale, comme des investigations préalables de l'impact du facteur.

17. Procédé selon l'une quelconque des revendications précédentes, dans lequel l'ajustement pour un ou plusieurs des facteurs séparés tient compte, dans l'ajustement, de l'effet variable du facteur à différentes occasions.

18. Procédé selon l'une quelconque des revendications précédentes, dans lequel l'ajustement, pour un ou plusieurs des facteurs séparés, tient compte, dans l'ajustement, de la nature aléatoire de l'ampleur de l'effet du facteur à différentes occasions.

19. Procédé selon l'une quelconque des revendications précédentes, dans lequel le premier profil de phase et/ou le profil modélisé est ajusté pour donner le profil de simulation, en effectuant un ajustement pour tenir compte des contributions relatives des deux sources ou plus à l'échantillon.

20. Procédé selon l'une quelconque des revendications précédentes, dans lequel l'indication de la vraisemblance du profil de données, au vu du génotype suggéré, est utilisée pour proposer un ou plusieurs génotypes de recherche parmi les génotypes suggérés, le ou les génotypes de recherche étant ces génotypes suggérés, pour lesquels l'indication de la vraisemblance du profil de données, au vu de ce génotype suggéré, est la plus élevée ou parmi les plus élevées.

21. Procédé selon la revendication 20, dans lequel le ou les génotypes de recherche sont enregistrés ou autrement entrés dans une base de données.

22. Procédé selon la revendication 20 ou 21, dans lequel le ou les génotypes de recherche sont étudiés contre le contenu d'une base de données, la recherche étant pour un ou plusieurs des génotypes de la base de données qui s'adaptent à un ou plusieurs génotypes de recherche.

23. Procédé selon la revendication 22, dans lequel la présence d'une adéquation entre un génotype de recherche et un génotype de base de données est utilisée comme indication d'un lien entre le génotype de recherche et le génotype de la base de données, par exemple que la source du génotype de recherche et du génotype de la base de donnée était la même personne.

24. Procédé selon l'une quelconque des revendications 20 à 23, dans lequel le ou les génotypes de recherche sont enregistrés ou autrement entrés dans la base de données, comme partie du processus de recherche et le ou les génotypes de recherche peuvent eux-mêmes être étudiés, comme des génotypes de base de données dans des recherches futures.

25. Procédé selon l'une quelconque des revendications 20 à 23, dans lequel le ou les génotypes de recherche sont soumis au processus de recherche, sans être enregistrés ou autrement entrés dans la base de données.

26. Procédé selon l'une quelconque des revendications précédentes, dans lequel l'indication est un rapport de vraisemblance et/ou une probabilité.

27. Procédé selon l'une quelconque des revendications précédentes, dans lequel l'indication comprend une évaluation de la fonction $p(d \mid A_i, w, H)$.

28. Procédé selon l'une quelconque des revendications précédentes, dans lequel la détermination du profil de données et/ou la suggestion du génotype suggéré et/ou la fourniture du premier profil de phase et/ou l'ajustement du profil modélisé et/ou l'ajustement au profil de simulation et/ou la comparaison et/ou l'indication sont effectués par un système expert et idéalement par un système automatisé.

29. Procédé selon l'une quelconque des revendications précédentes, dans lequel l'indication de la vraisemblance du profil de données, au vu du génotype suggéré, est de préférence utilisée pour déterminer le génotype attribué à cet échantillon, par exemple pour une recherche future et d'autres considérations.

30. Procédé selon l'une quelconque des revendications précédentes, dans lequel, lorsque l'indication de la vraisemblance satisfait des critères prédéterminés, ledit génotype suggéré est accepté comme représentant ledit échantillon.

FIG. 1

FIG. 2

FIG. 3

FIG. 4

FIG. 5

-2/2-

| LOCUS | SAMPLE SIZE | MEAN | STANDARD DEVIATION |
|---|---|---|---|
| D3 | 558 | 1.020 | 0.059 |
| VWA | 698 | 1.026 | 0.061 |
| D16 | 562 | 1.024 | 0.064 |
| D2 | 612 | 1.034 | 0.071 |
| D8 | 659 | 1.017 | 0.065 |
| D21 | 624 | 1.018 | 0.068 |
| D18 | 606 | 1.038 | 0.079 |
| D19 | 590 | 1.025 | 0.060 |
| THO1 | 648 | 1.001 | 0.071 |
| FGA | 569 | 1.027 | 0.070 |

## FIG. 6

| LOCUS | MEAN | STAN. DEV. | a | a |
|---|---|---|---|---|
| D3 | 0.0643 | 0.0161 | 14.9 | 216.3 |
| VWA | 0.0590 | 0.0165 | 12.0 | 191.0 |
| D16 | 0.0441 | 0.0151 | 8.1 | 175.8 |
| D2 | 0.0813 | 0.0265 | 8.6 | 96.8 |
| D8 | 0.0556 | 0.0225 | 5.7 | 97.0 |
| D21 | 0.0642 | 0.0242 | 6.5 | 95.1 |
| D18 | 0.0701 | 0.0258 | 6.8 | 90.1 |
| D19 | 0.0689 | 0.0191 | 12.0 | 162.8 |
| THO1 | 0.0539 | 0.0333 | 2.4 | 42.6 |
| FGA | 0.0591 | 0.0173 | 10.9 | 173.9 |

## FIG. 7

**EP 1 456 412 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 01007954 A

- GB 0130675 A

### Non-patent literature cited in the description

- **GILL et al.** Interpretation of simple mixtures of when artefacts such as stutters are present - with special reference to multiplex STRs used by the Forensic Science Service. *Forensic Science International,* 1998, vol. 95, 213-224

- Analysis And Interpretation Of Mixed Forensic Stains Using DNA STR Profiling. **CLAYTON et al.** Forensic Science International. Elsiever Scientific Publishers Ireland Ltd, vol. 91, 55-70